# EUROPÄISCHE PATENTSCHRIFT

(11) **EP 0 734 525 B1**
(45) Veröffentlichungstag und Bekanntmachung des Hinweises auf die Patenterteilung: **21.06.2000**
(21) Anmeldenummer: 95904481.9
(22) Anmeldetag: 15.12.1994
(51) Int. Cl.: G01N 33/50, C12Q 1/18, A01N 61/00

(54) **VERFAHREN ZUR IDENTIFIZIERUNG VON STOFFEN MIT POTENTIELLER HERBIZIDER ODER WACHSTUMSREGULATORISCHER WIRKUNG MITTELS PFLANZLICHER TRANSPORTERPROTEINE**
METHOD OF IDENTIFYING SUBSTANCES WITH A POTENTIAL HERBICIDAL OR GROWTH-REGULATORY ACTION BY MEANS OF VEGETABLE TRANSPORTER PROTEINS
PROCEDE UTILISANT DES PROTEINES VEGETALES TRANSPORTEUSES POUR L'IDENTIFICATION DE SUBSTANCES AYANT UN EFFET POTENTIEL HERBICIDE OU REGULATEUR DE CROISSANCE

(30) Priorität: 16.12.1993 DE 4343527
(43) Veröffentlichungstag der Anmeldung: 02.10.1996
(73) Patentinhaber: BASF AKTIENGESELLSCHAFT, 67056 Ludwigshafen (DE)
(72) Erfinder: FROMMER, Wolf-Bernd, D-14057 Berlin (DE); NINNEMANN, Olaf, D-10783 Berlin (DE); STREBER, Wolfgang, D-13509 Berlin (DE); RIESMEIER, Jörg, D-12163 Berlin (DE); KWART, Marion, D-12161 Berlin (DE)
(86) Internationale Anmeldenummer: EP9404174
(87) Internationale Veröffentlichungsnummer: WO9516913

(56) Entgegenhaltungen:
- WO-A-90/06047
- WO-A-94/00574
- WO-A-94/01559
- NATURE, Bd.330, 26. November 1987, LONDON GB Seiten 379 - 381 M. A. HEDIGER ET AL 'Expression cloning and cDNA sequencing of the Na+/glucose co-transporter.' in der Anmeldung erwähnt
- EMBO JOURNAL, Bd.13, Nr.15, 1. August 1994, EYNSHAM, OXFORD GB Seiten 3464 - 3471 O. NINNEMANN ET AL. 'Identification of a high affinity NH4+ transporter from plants.' in der Anmeldung erwähnt
- Cell, Vol.72(5), S.705-713, (1993)
- Eur.J.Med.Chem., Vol.24, S.415-420, (1989)
- J.Antibiotics, Vol.44, S.729-732, (1991)

## Beschreibung

Die vorliegende Erfindung betrifft Verfahren zur Identifizierung von Stoffen mit potentieller herbizider oder wachstumsregulatorischer Wirkung mittels pflanzlicher Transporterproteine (Transporter).

Von neuen Pflanzenschutzmitteln wird gefordert, daß sie gegenüber den herkömmlichen Mitteln eine geringere Toxizität, höhere Umweltverträglichkeit und verbesserte Wirksamkeit aufweisen. Die Erfolgswahrscheinlichkeit, mit der solche Stoffe gefunden werden, hängt neben der Zahl der zur Verfügung stehenden Stoffe wesentlich vom Verfahren ab, das zur Identifizierung der wirksamen Stoffe verwendet wird.
Da herkömmliche Verfahren meist darauf beruhen, daß Substanzen direkt an Pflanzen hinsichtlich ihrer herbiziden bzw. wachstumsregulatorischen Aktivität getestet werden, sind diese Verfahren in der Regel sehr zeit- und kostenintensiv und erfordern ausgedehnte Versuchsflächen. Folglich können vergleichsweise wenige Substanzen gleichzeitig getestet werden.

Es werden daher Verfahren benötigt, die sich einfach und schnell durchführen lassen, um eine ausreichende Anzahl von Stoffen untersuchen zu können.

Es wurde nun ein Verfahren gefunden, mit dem chemische Verbindungen identifiziert werden können, die spezifisch mit einem Transporterprotein aus Pflanzen interagieren. Das Verfahren besteht grundsätzlich darin, zunächst diese Stoffe an einem transgenen Organismus, vorzugsweise einem einzelligen Organismus, der ein pflanzliches Transporterprotein funktionell exprimiert, oder an transgenen Zellen, die ein pflanzliches Transporterprotein funktionell exprimieren, auf eine Hemmung des Transportvorgangs zu testen.

Die hierbei als inhibitorisch erkannten Stoffe werden anschließend an ganzen Pflanzen auf ihre Wirkung untersucht.
Unter dem Begriff Transporterproteine werden im Rahmen dieser Erfindung Proteine verstanden, die für den Transport von Stoffen über Membranen in pflanzlichen Zellen verantwortlich sind.
Bei dem Verfahren wird ein pflanzliches Transporterprotein in ein Testsystem integriert, das es ermöglicht, mit biochemischen, mikrobiologischen und physiologischen Meßmethoden die Funktion des Membrantransports qualitativ und quantitativ zu bestimmen. Die Verwendung dieses Testsystems ermöglicht das gezielte Auffinden von Wirkstoffen, die mit den Transportproteinen der Pflanze interagieren können. Die Interaktion von Stoffen mit einem Transporterprotein kann eine Hemmung oder Inaktivierung des Transportvorgangs bewirken, sie kann außerdem zum Transport des Stoffes selbst führen.
Da Transporivorgänge eine zentrale Rolle im Gesamtstoffwechsel von Pflanzen einnehmen und häufig essentiell für das Wachstum von Pflanzen sind, ermöglicht das erfindungsgemäße Verfahren, gezielt und mit einer hohen Wahrscheinlichkeit Substanzen zu identifizieren, die einen Einfluß auf das Pflanzenwachstum ausüben.
Die Interaktion kann eine wachstumsregulatorische bzw. herbizide Wirkung hervorrufen, wenn der aufgefundene Stoff zu einer Hemmung des natürlichen Transportvorgangs führt. Im Falle, daß der inhibitorisch wirksame Stoff selbst transportiert wird, kann er als Bestandteil von Pflanzenschutzmitteln wie Fungiziden, Insektiziden, Nematiziden und Akariziden, insbesondere Herbiziden und Wachstumsregulatoren, deren Mobilität in Pflanzen erhöhen und somit zu neuen wirksameren Mitteln führen.
Das erfindungsgemäße Teatsystem kann femer dazu verwendet werden, die pflanzlichen Transporterproteine auf molekularer Ebene zu untersuchen.

Es wurden bisher keine Verfahren zur Identifizierung von Substanzen mit herbizider oder wachstumsregulatorischer Wirkung mittels eines pflanzlichen Transporterproteins beschrieben. Ebenfalls nicht beschrieben ist die Hemmung pflanzlicher Transportvorgänge als Wirkungsmechanismus von Herbiziden. Ferner ist auch nicht bekannt, ob pflanzliche Transporterproteine aufgrund ihrer Stellung im Stoffwechsel, z.B. bei der Versorgung der Reproduktionsorgane ein interessantes potentielles Ziel ("target") für Herbizide bzw. Wachstumsregulatoren darstellen.

Bei dem Verfahren handelt es sich um ein biochemisches Testsystem, das in seiner ersten Stufe vorzugsweise an einzelligen Organismen oder an Zellen, die in Zellkultur gehalten werden, durchgeführt wird. Dieses Testsystem hat den Vorteil, daß es sich im Vergleich zu herkömmlichen Verfahren zur Identifizierung herbizider oder wachstumsregulatorischer Substanzen schnell und einfach durchführen läßt. Weiterhin ermöglicht es, bei geringem Zeitaufwand, eine Vielzahl von Substanzen hinsichtlich ihrer herbiziden oder wachstumsregulatorischen Wirkung zu untersuchen. Ferner bietet das erfindungsgemäße Verfahren den Vorteil, daß damit gezielt solche Stoffe identifiziert werden können, die mit einem ganz bestimmten pflanzlichen Protein interagieren. Um unerwünschte Wirkungen auf Mensch, Tier und Umwelt zu vermeiden, kann das Zielprotein ("target") in dem Verfahren so gewählt werden, daß seine Funktion für Pflanzen spezifisch ist.

Als Zielproteine werden in dem erfindungsgemäßen Verfahren bevorzugt Proteine eingesetzt, die in Pflanzen für den Transport von Substanzen über Membranen verantwortlich sind (Transporterproteine), vorzugsweise solche Transporterproteine, die spezifisch für Pflanzen sind.

Gegenstand der vorliegenden Erfindung ist somit ein Verfahren zur Identifizierung von Stoffen, welche eine potentielle herbizide oder wachstumsregulatorische Wirkung besitzen, die durch eine Hemmung oder Inaktivierung eines pflanzlichen Transportvorgangs zustande kommt, das dadurch gekennzeichnet ist, daß
a) zunächst das Transporterprotein durch heterologe Expression einer DNA-Sequenz, die für dieses Transportprotein kodiert, in einem transgenen Organismus oder transgenen Zellen hergestellt wird, anschließend
b) dieser rekombinante Organismus als Ganzes oder die transgenen Zellen zur Untersuchung einer chemischen Verbindung auf ihre inhibitorische Wirkung gegen das besagte Transporterprotein eingesetzt wird und
c) die Verbindung zusätzlich auf Aktivität gegenüber dem Organismus oder der Zelle untersucht wird, der oder die den entsprechenden Transporter nicht produziert, um die Möglichkeit auszuschließen, daß die Verbindung auch gegen andere Mechanismen dieses Organismus oder der Zelle inhibitorisch wirkt, und schließlich
d) die gegen den Transporter aktive Verbindung auf ihre herbizide oder wachstumsregulatorische Aktivität gegen Pflanzen geprüft wird.

Generell können bei dem erfindungsgemäßen Verfahren alle in Pflanzen vorkommenden Transporterproteine bzw. DNA-Sequenzen, die für diese Transporterproteine kodieren, verwendet werden.

Verschiedene Transporterproteine, die für den Transport von Substanzen über Membranen verantwortlich sind, sind in Pflanzen bereits identifiziert, z.T. stehen auch DNA-Sequenzen, die für derartige Transporterproteine kodieren, zur Verfügung.

So konnten beispielsweise Saccharosetransporter direkt an intakten Pflanzen bzw. an isoliertem Blattgewebe nachgewiesen werden. Die Saccharoseaufnahme ist dabei pH-abhängig (Giaquinta, Nature 267: 369-370, 1977, Annu. Rev. Plant Physiol. 34: 347-387, 1983; Delrot & Bonnemain, Plant Physiol. 67: 560-564, 1981; Delrot, Plant.Physiol. 67: 560-564, 1981). *p*-Chloromercuribenzylsulfonsäure und Diethylpyrocarbonat sind dabei hochwirksame Inhibitoren des Transportes (Bush, 1989, Plant Physiol. 89:1318-1323). cDNA-Sequenzen, die für pflanzliche Saccharosetransporter kodieren, sind bereits beschrieben, beispielsweise für Kartoffel (p62 bzw. StSUT1) und Spinat (S21 bzw. SoSUT1) (WO 94/00574; Riesmeier et al., 1993, Plant Cell 5:1591-1598; Riesmeier et al., 1992, EMBO J.11: 4705-4713), für *Arabidopsis thaliana* (suc1 und suc2-Gene; EMBL-Genbank: Zugriffsnr, X75365), *Plantago major* (EMBL-Genbank: Zugriffsnr. X75764), *L. esculentum* (EMBL-Genbank: Zugriffsnr. X82275) und *Nicotiana tabacum* (EMBL-Genbank: Zugriffsnr. X82276 und X82277). Im Fall der Saccharosetransporter gelang die Klonierung von cDNA-Sequenzen, die für diese Transporter kodieren, aus Spinat und Kartoffel durch Entwicklung eines artifiziellen Komplementationssystems in *Saccharomyces cerevisiae* (Riesmeier et al., EMBO J. 11: 4705-4713, 1992; Riesmeier et al., 1993, Plant Cell 5:1591-1598).

Aminosäuretransporter wurden ebenfalls bereits in Pflanzen identifiziert. Die den höheren Pflanzen verwandte Grünalge *Chlorella* verfügt über mindestens drei verschiedene, regulierte Aminosäure-Transportsysteme (Sauer und Tanner, Plant Physiol. 79: 760-764, 1985). Es handelt sich dabei um einen aktiven Transport, der über einen von der H⊕-ATPase generierten Protonengradienten energetisiert wird.
In höheren Pflanzen wurde indirekt durch Untersuchung von Xylem- und Phloemzusammensetzung auf die Existenz von einem passiven Transport (erleichterte Diffusion) geschlossen (Riens et al., Plant Physiol. 97: 227-233, 1991). Im Gegensatz dazu erfolgt die Beladung des Phloems bzw. Xylems von Ricinus-Kotyledonen, respektive Wurzeln, mit Aminosäuren selektiv und entgegen eines Konzentrationsgradienten (Schobert und Komor, Planta 177: 342-349,1989; Planta 181: 85-90, 1990). Die Existenz von mindestens vier unabhängigen H⊕-Kotransportern konnte an isolierten Vesikeln in verschiedenen Pflanzenarten nachgewiesen werden (Li und Bush, Plant Physiol. 94: 268-277. 1991).
Durch Komplementation einer Aminosäuretransport-Mutante der Hefe gelang es, Ureid- und Aminosäurepermease-Gene aus *Arabidopsis thaliana* zu isolieren und zu charakterisieren, beispielsweise cDNA-Sequenzen, die für die Aminosäuretransporter AAP1 und AAP2 kodieren (Frommer et al., 1993, Proc. Natl. Acad. Sei. USA 90:5944-5948; Kwart et al., 1993, Planta J. 4:993-1002; WO 94/01559).
Mit Hilfe eines Komplementationsverfahrens unter Verwendung der Hefemutante *shr3*, die nicht mehr in der Lage ist, endogene Aminosäuretransporter zur Zellmembran zu dirigieren (Ljungdahl et al., 1992, Cell 71:463-478), gelang es eine Reihe weiterer DNA-Sequenzen, die für Aminosäuretransporter aus Pflanzen kodieren, zu isolieren, z.B. cDNA-Sequenzen, die für die Aminosäuretransporter AAP3 (EMBL-Genbank, Zugriffsnummer:X77499), AAP4 (EMBL-Genbank, Zugriffsnummer:X77500), AAP5 (EMBL-Genbank, Zugriffsnummer:X77501), AAT1 (EMBL-Genbank, Zugriffsnummer:X71787) und NTR1 (EMBL-Genbank, ZugriffsnummerX77503) aus *Arabidopsis thaliana* kodieren.
Ferner sind cDNA-Sequenzen, die pflanzliche Ammoniumtransporter kodieren bekannt, beispielsweise eine für den Ammoniumtransporter AMT1 aus *Arabidopsis thaliana* kodierende cDNA (Ninnemann et al., 1994, EMBO J. 13:3464-3471; Deutsche Patentanmeldung P 43 37 597.9; EMBL-Genbank, Zugriffsnummer:X75879).

Die Klonierung von Genen membranständiger Transporterproteine wurde bereits neben den obengenannten Beispielen mehrfach beschrieben. Grundsätzlich können mehrere verschiedene Wege beschriften werden, um die Gene von Membranproteinen zu klonieren. Bei der Isolierung des Glucosetransportergens aus Erythrocyten gelang beispielsweise die Identifizierung von cDNA Klonen nach Reinigung des Proteins (Mueckler et al., Science 229: 941-945, 1985). Vielfach ist jedoch die Reinigung von Membrantransportem derart schwierig, daß andere Methoden genutzt werden müssen, z.B. die heterologe Expression in Oocyten (Hediger et al., Nature 330: 379-381, 1987). Pflanzliche Plasmalemma H⊕-ATPase-Gene wurden über Homologie zu tierischen und Pilz-Genen kloniert. Pflanzliche Glucosetransportergene konnten durch differentielles cDNA-Screening aus *Chlorella* isoliert werden (Sauer et al., EMBO J. 8: 3045-3050, 1990). Ein cDNA Klon aus *Chlorella* wurde als heterologe Probe genutzt, um mehrere Glucosetransportergene aus höheren Pflanzen zu klonieren (Sauer et al., 1990). Der chloroplastidäre Triosephosphat-Translokator (TPT) wurde über den Inhibitor DIDS radioaktiv markiert, das markierte Protein wurde gereinigt und ansequenziert. Abgeleitet von den partiellen Peptidsequenzen wurden synthetische Oligonukleotide als Proben zur Isolierung von TPT-kodierenden cDNAs benutzt (Flügge et al., EMBO J. 8: 39-46, 1989).
Die bereits bekannten DNA-Sequenzen, die für pflanzliche Transporterproteine kodieren, können ihrerseits zur Identifizierung und Isolierung weiterer DNA-Sequenzen, die für Transporterproteine kodieren, aus Pflanzen mittels gängiger molekularbiologischer Techniken verwendet werden.

In dem erfindungsgemäßen Verfahren werden bevorzugt Gene verwendet, die für Transporterproteine kodieren, die essentiell für das Wachstum der Pflanze sind. Eine Inhibition des entsprechenden Transporterproteins sollte demnach zu einer Beeinträchtigung des Wachstums führen. Unter der Voraussetzung, daß DNA-Sequenzen, die für das entsprechende Transporterprotein kodieren, verfügbar sind, läßt sich dieser Nachweis beispielsweise mittels der Expression oder der *anti-sense*-Inhibition des entsprechenden Gens in transgenen Pflanzen erbringen (Willmitzer, Trends Genet. 4:13-18, 1988). Die Techniken zur Herstellung derartiger transgener Pflanzen sind dem Fachmann bekannt. So konnte durch Expression einer *anti-sense*-RNA, die für den Triosephosphat-Translokator kodiert, beispielsweise gezeigt werden, daß bereits eine geringe Reduktion der Expression des Proteins zu einer drastischen Wachstumshemmung der Pflanze führt (Riesmeier et al., Proc. Natl. Acad. Sci. USA 90: 6160-6164, 1993). Ebenso konnte für den Saccharosetransporter gezeigt werden, daß eine Reduktion der Aktivität zu einer starken Wachstumshemmung bei Kartoffelpflanzen führt. Ferner sind bei den betroffenen Pflanzen die Blätter geschädigt und die Pflanzen bilden wenig bis keine Kartoffelknollen mehr (Riesmeier et al., 1994, EMBO J. 13:1-7). Da die Bildung von Reproduktionsorganen stark beeinträchtigt wird, kann erwartet werden, daß ein geeignetes Herbizid nicht nur das Wachstum einer Pflanze hemmt, sondern auch ihre Vermehrung verhindert. Gleiches ist im Fall des Ammonium- und Aminosäuretransports zu erwarten, da diese im Stoffwechsel eine essentielle Funktion für den Transport von Stickstoff einnehmen.

Aufgrund der beschriebenen essentiellen Funktion sehen bevorzugte Ausführungsformen der vorliegenden Erfindung die Verwendung der Transporterproteine für Aminosäuren, Saccharose und Ammonium als Zielproteine für das Auffinden potentieller pflanzenspezifischer Wirkstoffe vor, insbesondere die Verwendung der oben im einzelnen genannten Transporterproteine.

Geeignete Gene, die für Transporterproteine kodieren, werden mit Hilfe gängiger molekulargenetischer Verfahren derartig in einen Organismus oder in Zellen eingebracht, daß die Expression eines funktionsfähigen Transporterproteins gewährleistet ist.

Bei dem unter Verfahrensschritt a) genannten Organismus handelt es sich vorzugsweise um einen einzelligen Organismus. Der einzellige Organismus wird so gewählt, daß seine Zellen leicht kultivierbar und für die Expression des Transporterproteins geeignet sind. Besonders geeignet für diesen Zweck sind Mikroorganismen wie Bakterien, Pilze oder Hefen. Es können jedoch auch einzelne Zellen eines Organismus verwendet werden. Geeignet für die Verwendung in dem erfindungsgemäßen Verfahren sind beispielsweise auch in Zellkultur gehaltene pflanzliche Zellen oder Kalluskulturen, ebenso wie tierische Zellen in Zellkultur, insbesondere auch Oozyten, vorzugsweise *Xenopus-*Oozyten.
Die durch die Einführung eines pflanzlichen Transporterprotein-Gens erhaltenen transgenen Zellen bzw. der erhaltene rekombinante Organismus können dann als Ganzes Bestandteil eines Testsystems sein, oder können zur Isolierung von Membranen oder gereinigtem Transporterprotein verwendet werden. Diese rekombinanten Organismen (Bakterien, Pilze und Hefen) und transgenen Zellen sind ebenfalls Gegenstand der Erfindung.
Ein Gen, das für ein Transporterprotein kodiert, kann außerdem in eine bestimmte Mutante eines Organismus eingebracht werden, die ohne die Funktion des entsprechenden Transporterproteins nicht wachstumsfähig ist (Riesmeier et al. EMBO J. 11: 4705-4713, 1992). Die Verwendung einer solchen Mutante hat den besonderen Vorteil, daß dadurch das Wachstum des rekombinanten Organismus in einem geeignetem Medium als Maß für die Funktion des Transporters gelten kann und somit den Einfluß von zu untersuchenden Stoffen auf den Membrantransport quantitativ beschreiben kann. Der Wachstumstest zeichnet sich durch eine besonders einfache Handhabung und den schnellen Durchsatz von Stoffen aus. Bevorzugt ist daher die Verwendung geeigneter Mutanten, wie sie z. B. für Saccharosetransporter (SUSY7; siehe Riesmeier et al. EMBO J. 11: 4705-4713, 1992), Aminosäuretransporter (Hefemutanten 22574d und JT16; siehe Frommer et al. Proc. Natl. Acat. Sci. USA 90: 5944-5948, 1993; Hefemutante *shr3*; siehe Ljungdahl et al., 1992, Cell 71:463-478) und Ammoniumtransporter (Ninnemann et al., 1994, EMBO J. 13:3464-3471) beschrieben wurden.

Der besagte Organismus bzw. Zellen werden mit dem entsprechenden Transportergen transformiert. Der rekombinante Organismus bzw. die transgenen Zellen können anschließend mit gängigen mikrobiologischen Methoden beliebig vermehrt werden und stehen damit in unbegrenztem Ausmaß für den Einsatz in dem Testsystem zur Verfügung. Zum Testen von Stoffen auf herbizide und/oder wachstumsregulatorische Eigenschaften wird eine mehrstufige Vorgehensweise bevorzugt: Zunächst werden rekombinante Organismen bzw. transgene Zellen in einem Medium angezogen, bei dem ein essentielles Wachstumssubstrat so gewählt ist, daß es nur durch den zu untersuchenden Transporter in die Zellen gelangt und außerdem von keinem anderen Substrat des Mediums funktionell ersetzt werden kann. Im Falle eines Saccharosetransporters befindet sich z.B. im Medium Saccharose als einzige Kohlenstoffquelle, im Falle eines Aminosäuretransporters befindet sich im Medium z.B. eine Aminosäure, die den Zellen als einzige Kohlenstoff- oder Stickstoffquelle dient, und im Falle eines Ammoniumtransporters befindet sich als einzige Stickstoffquelle Ammonium im Medium. Die zu untersuchenden Stoffe werden dem Medium während der Wachstumsphase zugesetzt und das Wachstum der Zellen wird mit gängigen Methoden bestimmt.

Um eine spezifische Interaktion eines Stoffes mit einem Membrantransporter nachzuweisen und um andere Wirkungsweisen als Ursache der Wachstumshemmung auszuschließen, muß der Stoff folgende Bedingungen erfüllen:
1) Rekombinante Zellen müssen nach Zusatz des Stoffes deutlich geringeres Wachstum zeigen als ohne Zusatz des Stoffes.
2) Die gleichen Organismen dürfen nach Zusatz des Stoffes im Wachstum nicht gehemmt sein, wenn ein anderes Wachstumssubstrat im Medium enthalten ist, welches das eigentliche Substrat des Transporters in den Zellen funktionell ersetzen kann und selbst nicht über den entsprechenden Transporter in die Zellen gelangt.

Hefezellen können z.B. alternativ mit Saccharose oder mit einer Aminosäure als Kohlenstoffquelle wachsen. Als Stickstoffquelle können Hefen alternativ eine Aminosäure oder Ammoniumionen benützen.

Erfüllt ein Stoff die genannten Bedingungen, so kann er in einem biochemischen Test weiter untersucht werden. Bei diesem Test wird der Membrandurchtritt des natürlichen Substrates oder des inhibierenden Stoffes gemessen. Dazu können ganze Hefezellen oder isolierte Membranvesikel eingesetzt werden. Der Membrandurchtritt des Transportersubstrates oder des inhibierenden Stoffes kann nachgewiesen werden, indem das Substrat oder der Stoff aus den abgetrennten Zellen oder Membranvesikeln isoliert und mit gängigen analytischen Verfahren nachgewiesen wird. Im Regelfall wird das Substrat oder der Stoff in radioaktiv markierter Form zugesetzt und später über seine radioaktive Strahlung analysiert. Durch Variation der Konzentrationen des Substrates und des inhibierenden Stoffes kann mit gängigen biochemischen Verfahren der Typ der Hemmung bestimmt werden. Des weiteren kann damit nachgewiesen werden, ob der Stoff selbst durch den Transporter transportiert wird.

Erfüllt ein Stoff die obengenannten Bedingungen, so kann er mit oder ohne weitere biochemische Untersuchungen direkt an ganzen Pflanzen oder geeigneten Pflanzenteilen auf seine herbizide Wirkung oder auf seine Mobilität in der Pflanze untersucht werden. Dazu können die gängigen herbologischen und physiologischen Verfahren angewandt werden.

Die über das erfindungsgemäße Verfahren identifizierbaren herbiziden und/oder wachstumsregulatorischen Wirkstoffe können alleine oder in Verbindung mit anderen Herbiziden, Wachstumsregulatoren, Nematiziden, Insektiziden, Akariziden, Fungiziden und in der Landwirtschaft üblichen Hilfsstoffen als Pflanzenschutzmittel eingesetzt werden.

Mit dem Verfahren können auch Stoffe identifiziert werden, welche selbst von dem Transporterprotein (Transporter) durch die pflanzliche Zellmembran geschleust werden.

Das Verfahren kann damit zur Identifizierung solcher chemischen Strukturen dienen, welche in der Pflanze besonders gut transportiert werden. Diese Eigenschaft der guten Mobilität ist für Pflanzenschutzmittel wie Herbizide und Insektizide besonders erwünscht.

Das Verfahren läßt sich ferner als Testsystem zur Identifizierung transportierbarer chemischer Strukturen verwenden. Da Saccharose- und Aminosäuretransporter die Haupttransportsysteme für organische Moleküle in den Membranen darstellen, kann beispielsweise das Hefesystem als einfacher Test genutzt werden, um die Mobilität von organischen Substanzen in der Pflanze zu untersuchen. Bevorzugt wird dabei eine mehrstufige Vorgehensweise: Zunächst kann in einem Wachstumstest die Aufnahme von fremden Stoffen untersucht werden. Darauf aufbauend können dann direkte Transportmessungen an intakten Hefen oder an isolierten Membranen zur genaueren Untersuchung herangezogen werden. Als Kontrolle stehen immer Hefen zur Verfügung, die den Transporter nicht enthalten. Neben Hefen können hierfür auch andere Organismen, insbesondere einzellige Organismen, und insbesondere auch Zellkulturen tierischer oder pflanzlicher Zellen verwendet werden.

Im Fall einer spezifischen Beeinträchtigung des Transports sollte ein verringertes Wachstum in den transgenen Hefen beobachtet werden. Man kann durch Untersuchung der Transporter zu einem verbesserten Verständnis kommen, welche Substanzeigenschaften für eine Transportierbarkeit notwendig sind. Für eine Reihe von Stoffen, die bisher als Pestizide aufgrund fehlender Transportierbarkeit unwirksam waren, wäre es möglich, die Moleküle chemisch so zu verändern, daß sie diese Substanzeigenschaften erhalten und dadurch besser zu ihrem Wirkort gelangen. Ebenfalls wäre es denkbar, die Transporter mittels molekularbiologischer Methoden oder durch Mutation so zu verändern, daß sie die Pestizide wie Insektizide und Herbizide besser durch Membranen transportieren können. Die veränderten Transporterprotein-Gene können dann wiederum in Pflanzen eingebracht werden.

Das System kann neben dem Auffinden von Substanzen auch zur Untersuchung anderer wichtiger Fragen eingesetzt werden, z.B. wie derartige Proteine strukturell aufgebaut sind und wie die Substrate erkannt werden, bzw. und in welchem Zusammenhang die Struktur zur Funktion in Beziehung steht und wie generell Stoffe in einem Organismus verteilt werden. Die Methode, die zur Isolierung und Charakerisierung der beschriebenen Transporter geführt hat, kann aber auch eingesetzt werden, um weitere Proteine (z.B. Ionentransporter oder Transportproteine aus tierischen Systemen) zu isolieren und auf die gleiche Weise zu untersuchen.

Die nachfolgenden Anwendungsbeispiele erläutern den Gegenstand der Erfindung ohne ihn einzuschränken. Es wird die Verwendung von pflanzlichen Saccharose-, Aminosäure- bzw. Ammoniumtransportern zur Identifizierung von Substanzen, die auf diese Transporter inhibitorisch wirken, beschrieben.

### Anwendungebeispiele

### Beispiel 1

### Identifizierung von Inhibitoren pflanzlicher Aminosäuretransporter

Für die Identifizierung von Inhibitoren von pflanzlichen Aminosäuretransportern werden Transportuntersuchungen an der Hefemutante 22574d (Jauniaux & Grenson, Eur. J. Biochem. 190: 39-44, 1990), die eine Mutation in dem Gen der Aminosäurepermease trägt, bzw. an der Hefemutante JT 16 (Tanaka und Fink, Gene 38: 205-214, 1985), die nicht in der Lage ist, Histidin aufzunehmen, durchgeführt.
Diese Mutanten werden mit cDNA-Sequenzen, die für pflanzliche Aminosäuretransporter kodieren und zur Expression funktioneller Transporter in den Hefezellen führen, transformiert. Bei den cDNA-Sequenzen handelt es sich um cDNA-Sequenzen, die für die Aminosäuretransporter AAP1 bzw. AAP2 aus *Arabidopsis thaliana* kodieren (Frommer et al., 1993, Proc. Natl. Acad. Sci. USA 90:5944-5948; Kwart et al., 1993, Plant J. 4:993-1002).
Für die Messungen werden Zellen dieser Mutanten bei 28°C in Minimalmedium (NAAG + 5 mmol/l Prolin) angezogen und in logarithmischer Phase (OD₆₀₀=0,6) geerntet. Die Zelten werden 10 Minuten bei 4000 rpm und 4 Grad Celsius in einer Zentrifuge abzentrifugiert und 2x mit AUB-Puffer (modifizierter AAB-Puffer, Ljungdahl et al. ) gewaschen. Die Zellenkonzentration wird auf eine OD₆₀₀ von 25 in AUB-Puffer eingestellt.

Verwendete Medien und Puffer:
- NAAG-Medium:: - 1,7 g/l Hefe-Nitrogenbase, w/o Aminosäuren
- und: Ammoniumsulfat
- SDGlu-Medium:: - 10 g/l Glucose - 20 g/l Agarose - 6,7 g/ Hefe-Nitrogenbase w/o Aminosäuren
- SDsuc-Medium:: - 20 g/l Gukose - 20 g/l Agarose - 6,7 g/ Hefe-Nitrogenbase w/o Aminosäuren -20 g/l Saccharose -20 g/l Agarose
- AUB-Puffer:: - 10 mmol/l MES - 2 mmol/l Magnesiumchlorid - 0,6 mmol/l Sorbitol

Jeweils 100 µl der Zellsuspension werden mit 100 µl einer 1 mmol/l L-Prolin-Lösung (18,5 kBq L-[14C]- Prolin) und verschiedenen Konzentrationen der entsprechenden zu untersuchenden Substanz versetzt. Nach 20, 60, 120 und 180 Sekunden wird ein 50 µl-Aliquot abgenommen, in 4 ml eiskaltem AUB-Puffer verdünnt und über Glasfieberfilter abfiltriert. Um unspezifisch gebundenes L-[14C]-Prolin zu entfernen, werden die Zellen 2x mit jeweils 4 ml eiskaltem Wasser gewaschen. Die im Glasfilter zurückgehaltene Radioaktivität wird anschließend im Flüssigkeitsszintillationszähler gemessen. Die Radioaktivität spiegelt die von den Zellen aufgenommene Menge an radioaktiv markiertem Prolin wieder.
Als Kontrolle wird zum einen die Aufnahme radioaktiv markierten Prolins ohne Zusatz eines potentiellen Inhibitors bestimmt.
Ferner werden als Kontrolle jeweils Zellen der Mutanten 22574d bzw. JT 16 verwendet, die nicht mit cDNA-Sequenzen, die für pflanzliche Aminosäuretransporter kodieren, transformiert waren.
Ergebisse derartiger Messungen an der Mutanten 22457d-AAP2, die den Aminosäuretransporter AAP2 aus *Arabisopsis thaliana* exprimiert, sind in der folgenden Tabelle dargestellt.

| getestete Substanz | Konzentration | Aktivität relativ zur Kontrolle [%] |
|---|---|---|
| CCCP | 10 µM | 15,6 ± 2,1 |
| DNP | 0,1 mM | 7,6 ± 1,6 |
| DEPC | 1 mM | 3,1 ± 1,2 |
| Azetidin-2-Carboxylat | 10 mM | 62 |
| D-Prolin | 10 mM | 90 |

Für die Untersuchungen wurden Hefezellen des Stammes 22457d mit einer cDNA-Sequenz, die für den Aminosäuretransporter AAP2 kodiert, transformiert und die Aufnahme radioaktiv markierten Prolins in Gegenwart verschiedener Substanzen bestimmt.
Die verschiedenen getesteten Substanzen, deren Konzentration, sowie die Aktivität des Transporters, dargestellt als prozentuale Aktivität der Kontrolle, bei der keine potentiellen Inhibitoren zugesetzt wurden, sind angegeben.

Substanzen, die spezifisch eine inhibitorische Wirkung auf den Aminosäuretransporter AAP2 zeigen, werden an ganzen Pflanzen auf ihre herbizide bzw. wachstumsregulatorische Wirkung getestet.

### Beispiel 2

### Identifizierung von Inhibitoren des Saccharosetransporters aus Spinacia oleracea

Für die Identifizierung von Substanzen, die eine inhibitorische Wirkung auf Saccharosetransporter aus Spinat besitzen, werden Hefezellen des Stammes SUSY7 (Riesmeier et al., 1992, EMBO J. 11:4705-4713) verwendet. Zellen dieses Stammes werden mit einer cDNA-Sequenz, die für den Saccharosetransporter S21 (SoSUT1) aus Spinat kodiert und die Expression eines funktionellen Transporters in den Zellen gewährleistet, transformiert.
Die Hefezellen werden in Minimalmedium (SD + 2% (w/v) Saccharose, pH 3,8) bei 28°C angezogen und in logarithmischer Phase (OD₆₀₀=0,6) geerntet. Die Zellen werden 10 Minuten bei 4000 rpm abzentrifugiert und 2mal mit SD-Medium gewaschen. Gemäß dem Naßgewicht der Zellen wird eine Zellkonzentration von c = 50 g/l in SD-Medium eingestellt und Fraktionen von 200 µl aliquotiert. Vor der eigentlichen Reaktion werden die Zellen für 5 min in 10 mM Glucose inkubiert und mit MES-Puffer auf pH 3,8 eingestellt. Die Reaktion wird durch Zugabe von 200 µl einer 0,2 mmol/l [14C]-Saccharoselösung (3 mCi/mmol) in SD-Medium (pH 3,8) gestartet. Nach 20, 60, 120, 180 und 240 Sekunden werden jeweils 70 µl der Suspension abgenommen und in 4 ml eiskaltes Wasser pipettiert, um die Reaktion abzubrechen. Die Zellen werden über Glasfiberfilter abfiltriert und zweimal mit eiskaltem Wasser gewaschen. Die Radioaktivität auf den Filtern wird anschließend im Flüssigkeitsszintillationszähler bestimmt. Die zu testenden Substanzen werden 30 Sekunden vor der Zugabe der Saccharose zugegeben.
Als Kontrolle wird zum einen die Aufnahme radioaktiv markierter Saccharose ohne Zusatz eines potentiellen Inhibitors bestimmt.
Ferner dienen als Kontrolle Zelten des Stammes SUSY7, die nicht mit cDNA-Sequenzen, die für einen Saccharosetransporter aus Spinat kodieren, transformiert werden.
Die Ergebnisse derartiger Transportmessungen sind in der folgenden Tabelle dargestellt.

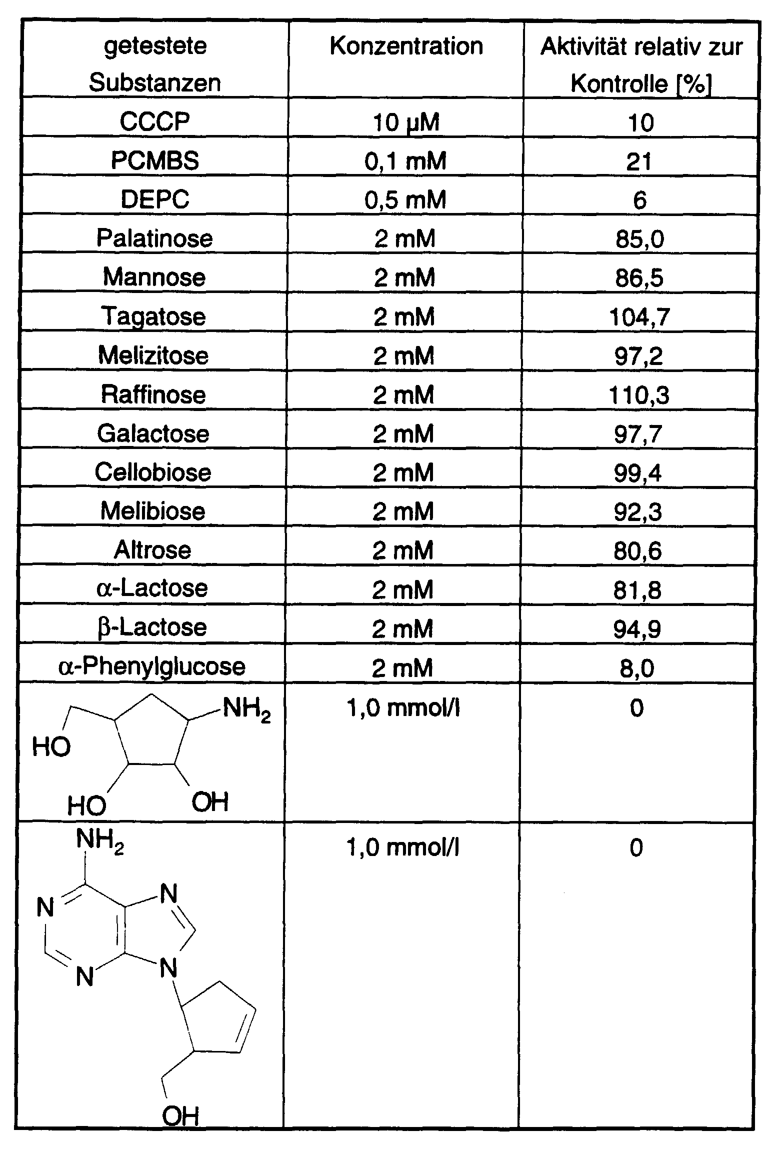

Die verschiedenen getesteten Substanzen, deren Konzentration, sowie die Aktivität des Transporters, dargestellt als prozentuale Aktivität der Kontrolle, bei der keine potentiellen Inhibitoren zugesetzt wurden, sind angegeben. Substanzen, die spezifisch eine inhibitorische Wirkung auf den Saccharosetransporter S21 (SoSUT1) zeigen, werden an ganzen Pflanzen auf ihre herbizide bzw. wachstumsregulatorische Wirkung getestet.
Von den identifizierten Inhibitoren des Saccharosetransporters wirken einige herbizid auf Pflanzen, beispielsweise PCMBS.

### Beispiel 3

### Identifizierung von Inhibitoren des Saccharosetransporters aus Solanum tuberosum

Für die Identifizierung von Substanzen, die eine inhibitorische Wirkung auf Saccharosetransporter aus Kartoffel besitzen, werden Hefezellen des Stammes SUSY7 (Riesmeier et al., 1992, EMBO J. 11:4705-4713) verwendet. Zellen dieses Stammes werden mit einer cDNA-Sequenz, die für den Saccharosetransporter P62 (StSUT1) aus Kartoffel kodiert (WO 94/00574, Riesmeier et al., 1993, Plant Cell 5:1591-1598) und die Expression eines funktionellen Transporters in den Zellen gewährleistet, transformiert. Der resultierende Hefestamm wurde SUSY-7-P62 (StSUT1) genannt.
Die Hefezellen werden in Minimalmedium (SD + 2% (w/v) Saccharose, pH 3,8) bei 28°C angezogen und in logarithmischer Phase (OD₆₀₀=0,6) geerntet. Die Zellen werden 10 Minuten bei 4000 rpm abzentrifugiert und 2mal mit SD-Medium gewaschen. Gemäß dem Naßgewicht der Zellen wird eine Zellkonzentration von c = 50 g/l in SD-Medium eingestellt und Fraktionen von 200 µl aliquotiert. Vor der eigentlichen Reaktion werden die Zellen für 5 min in 10 mM Glucose inkubiert und mit MES-Puffer auf pH 3,8 eingestellt. Die Reaktion wird durch Zugabe von 200 µl einer 0,2 mmol/l [14C]-Saccharoselösung (3 mCi/mmol) in SD-Medium (pH 3,8) gestartet. Nach 20, 60, 120, 180 und 240 Sekunden werden jeweils 70 µl der Suspension abgenommen und in 4 ml eiskaltes Wasser pipettiert, um die Reaktion abzubrechen. Die Zellen werden über Glasfiberfilter abfiltriert und zweimal mit eiskaltem Wasser gewaschen. Die Radioaktivität auf den Filtern wird anschließend im Flüssigkeitsszintillationszähler bestimmt. Die zu testenden Substanzen werden 30 Sekunden vor der Zugabe der Saccharose zugegeben.

Als Kontrolle wird zum einen die Aufnahme radioaktiv markierter Saccharose ohne Zusatz eines potentiellen Inhibitors bestimmt.
Ferner dienen als Kontrolle Zellen des Stammes SUSY7, die nicht mit cDNA-Sequenzen, die für einen Saccharosetransporter aus Kartoffel kodieren, transformiert werden.
Die Ergebnisse derartiger Transportmessungen sind in der folgenden Tabelle dargestellt.

| getestete Substanzen | Konzentration | Aktivität relativ zur Kontrolle [%] |
|---|---|---|
| CCCP | 10 µM | 9 |
| PCMBS | 0,1 mM | 20 |
| 2,4-DNP | 0,1 mM | 3 |
| DEPC | 0,5 mM | 6 |
| N-Ethylmaleimid | 1 mM | 22 |
| Palatinose | 2 mM | 102 |
| Tagatose | 2 mM | 103 |
| Raffinose | 2 mM | 110 |
| β-Lactose | 2 mM | 91 |
| α-Phenylglukose | 2 mM | 7 |

Die verschiedenen getesteten Substanzen, deren Konzentration, sowie die Aktivität des Transporters, dargestellt als prozentuale Aktivität der Kontrolle, bei der keine potentiellen Inhibitoren zugesetzt wurden, sind angegeben.

Substanzen, die spezifisch eine inhibitorische Wirkung auf den Saccharosetransporter P62 (StSUT1) zeigen, werden an ganzen Pflanzen auf ihre herbizide bzw. wachstumsregulatorische Wirkung getestet.
Von den identifizierten Inhibitoren des Saccharosetransporters wirken einige herbizid auf Pflanzen, beispielsweise PCMBS.

### Beispiel 4

### Bestimmung der Substratspezifität des Saccharosetransporters

Die Untersuchungen zur Substratspezifität und die Kₘ-Wert-Bestimmung werden im Hefestamm SUSY7-pP62 (StSUT1) (Riesmeier et al., 1993, Plant Cell 5:1591-1598), der den Saccharosetransporter aus *Solanum tuberosum* exprimiert, durchgeführt. Dabei wird wie in Beispiel 3 verfahren, ausgenommen daß die Substratkonzentration verändert wird und keine potentiellen Inhibitoren zugesetzt werden. Zur Kontrolle der Hintergrundaktivität wird die Aufnahme von [14C]-Saccharose durch den Hefe-Stamm SUSY7 bestimmt, der den Transporter nicht exprimiert. Dieser Stamm zeigt über den Zeitraum der Messung keine meßbare Aufnahme von [14C]-Saccharose. Versuchsergebnisse:
- Kₘ für Saccharose: 1 mM
- Kₘ für Maltose: 10 mM

### Beispiel 5

### Aktivierung des Saccharosetransports

Der Saccharosetransport des Saccharosetransporter P62 (StSUT1) aus *Solanum tuberosum* kann durch eine vorherige Energetisierung der Hefezellen durch Inkubation in Glukose, Stachyose und Adenin gesteigert werden. Die gleiche Erhöhung der Transportaktivität wird durch eine Verringerung des pH-Werts auf pH 3,8 in der Messung erzielt.
Die Transportmessung zur Bestimmung der Aktivierung erfolgt wie in Beispiel 3 beschrieben, jedoch werden die Zellen vor der eigentlichen Messung nicht für 5 min in 10 mM Glucose, sondern für 5 min in Lösungen verschiedener Konzentrationen an Glucose, Stachyose bzw. Adenin inkubiert. Als Kontrolle dienen Hefezellen, die vor der Messung nicht in Lösungen dieser Substanzen inkubiert werden. Die folgende Tabelle gibt das Ausmaß der Aktivierung des Saccharose-transports bei verschiedenen Konzentrationen von Glucose, Stachyose bzw. Adenin im Vergleich zur Kontrolle an.

| | Aktivität relativ zur Kontrolle [%] | |
|---|---|---|
| | c1 [0,2 mmol/l] | c2 [2 mmol/l] |
| Glucose | 89 ± 5 | 90 ± 3 |
| Stachyose | 144 ± 9 | 101 ± 8 |
| Adenin | 141 ± 5 | 225 ± 6 |

Stachyose stimuliert bei geringen Konzentrationen den Saccharosetransport, bei hohen Konzentrazionen (c > 0,4 mmol/l) kehrt sich dieser Effekt um und Stachyose beginnt die Aufnahme zu verringern. Für Adenin besteht ein linearer Zusammenhang zwischen der Adeninkonzentration und der Erhöhung der Saccharosetransportrate. Durch Kompetitionsstudien kann gezeigt werden, welche Domänen für die Affinität des Transporters zu Saccharose wichtig sind.

### Beispiel 6

### Identifizierung von Inhibitoren pflanzlicher Ammoniumtransporter

Die Transportmessungen zur Identifizierung von Inhibitoren pflanzlicher Ammoniumtransporter werden mit dem Strukturanalogon Methylamin durchgeführt, da radioaktiv markiertes Ammonium kommerziell nicht erhältlich ist.

Für die Untersuchungen werden Hefen des Hefestammes Σ 26972c (Dubois & Grenson, Mol. Gen. Genet. 175: 67-76, 1979) verwendet, der Mutationen in den NH₄⁺-Permease-Genen MEP1 und MEP2 trägt.

Die Hefezellen werden nach Standardmethoden mit cDNA-Sequenzen, die für einen Ammoniumtransporter aus Pflanzen kodieren und die Expression eines funktionellen Transporters in den Hefezellen erlauben, transformiert. Es handelt sich dabei um den Transporter AMT1 aus *Arabidopsis thaliana* (Ninnemann et al., 1994, EMBO J. 13:3464-3471). Die Zellen werden in NAAG-Medium (2 % Glucose, 1,7 g/l "Yeast nitrogen-base w/o amino acids and ammoniumsulfate" (Difco), angereichert mit 500 µg/ml L-Prolin), bei 28 Grad Celsius angezogen und in der logarithmischen Phase geerntet (OD₆₆₀.=0,6). Anschließend werden die Zellen 10 Minuten bei 4000 rpm und 4 Grad Celsius in einer Zentrifuge abzentrifugiert, 2mal mit 20 mM Natriumphosphatpuffer, pH 7, gewaschen und in demselben Puffer zu einer OD₆₆₀ von 8 aufgenommen. Die Zellsuspension wird in Fraktionen von 200µl aliquotiert. 5 Minuten vor der eigentlichen Messung werden die Hefen durch Zugabe von 100 mM Glucose aktiviert und bei 30 Grad Celsius inkubiert. Zum Start der Reaktion werden 100 µl der Zellsuspension zu 100µl Reaktionsgemisch (20 mM Na-Phosphatpuffer, pH7; 18,5 kBq [¹⁴C]-Methylamin (NEN); 100µM Methylamin; zu testende Substanz je nach Experiment (siehe Fig. 4)) gegeben. Jeweils 10, 60, 120 und 180 Sekunden nach Start der Reaktion werden 50 µl Aliquots entnommen, in 4 ml eiskalte 5mM Methylamin-Lösung gegeben und auf Glasfaserfilter filtriert. Nach Waschen der Filter mit weiteren 8 ml der Methylaminlösung wird die Radioaktivität auf den Filtern anschließend im Flüssigkeitsszintillationszähler bestimmt. Inhibitoren werden 60 Sekunden vor Zugabe der Glucose zugegeben.

Als Kontrolle wird zum einen die Aufnahme radioaktiv markierten Methylamins ohne Zusatz eines potentiellen Inhibitors bestimmt. Ferner werden als Kontrolle dieselben Transportuntersuchungen an Hefezellen des Stammes Σ 26972c durchgeführt, die nicht transformiert wurden.

Die Ergebnisse derartiger Untersuchungen sind in der folgenden Tabelle dargestellt.

| getestete Substanzen | Konzentration | Aktivität relativ zur Kontrolle [%] |
|---|---|---|
| Keine | / | 100 |
| Methylamin | 500µM | 40 |
| Dimethylamin | 500µM | 89 |
| Trimethylamin | 500µM | 89 |
| Ethylamin | 500µM | 93 |
| KCl | 500µM | 98 |
| RbCl | 500µM | 96 |
| CsCl | 500µM | 98 |
| NH₄Cl | 500µM | 10 |
| Cycloheximid | 10µg/ml | 85 |
| Antimycin A | 10µg/ml | 8 |
| DCCD | 200µM | 15 |
| 2-4 DNP | 100µM | 18 |
| CCCP | 10µM | 40 |

Die verschiedenen getesteten Substanzen, deren Konzentration, sowie die Aktivität des Transporters, dargestellt als prozentuale Aktivität der Kontrolle, bei der keine potentiellen Inhibitoren zugesetzt wurden, sind angegeben.

Substanzen, die spezifisch eine inhibitorische Wirkung auf den Ammoniumtransporter AMT1 zeigen, werden an ganzen Pflanzen auf ihre herbizide bzw. wachstumsregulatorische Wirkung getestet.

Von den identifizierten Inhibitoren des untersuchten Ammoniumtransporters wirken einige herbizid auf Pflanzen, beispielsweise Methylamin.

### Beispiel 7

### Bestimmung der Substratspezifität des Ammoniumtransporters AMT1 aus Arabidopsis thaliana

Die Untersuchungen zur Substratspezifität und die Kₘ-Wert-Bestimmungen werden mit dem Hefestamm Σ 26972c (Dubois & Grenson, Mol. Gen. Genet. 175: 67-76, 1979), der den pflanzlichen Ammoniumtransporter AMT1 exprimiert, vorgenommen. Dabei wird wie in Beispiel 6 verfahren, ausgenommen, daß die Methylamin-Substratkonzentration variiert wird. Zur Kontrolle der Hintergrundaktivität wird die Aufnahme von [¹⁴C]-Methylamin durch einen Hefestamm bestimmt, der den besagten Transporter nicht exprimiert. Zur Bestimmung der Affinität des Transportsystems für Ammonium wird die Inhibition des Methylamintransports durch verschiedene Konzentration von Ammonium bestimmt (Inhibitorkonstante, Kⱼ).
- Kₘ für Methylamin: 65 µM
- Kⱼ für Ammonium: <10µM

## Patentansprüche

1. Verfahren zur Identifizierung von Stoffen, welche eine potentielle herbizide oder wachstumsregulatorische Wirkung besitzen, die durch eine Hemmung oder Inaktivierung eines pflanzlichen Transportvorgangs zustande kommt, dadurch gekennzeichnet, daß
a) zunächst das Transporterprotein durch heterologe Expression einer DNA-Sequenz, die für dieses Transportprotein kodiert, in einem transgenen Organismus oder transgenen Zellen hergestellt wird, anschließend
b) dieser rekombinante Organismus als Ganzes oder die transgenen Zellen zur Untersuchung einer chemischen Verbindung auf ihre inhibitorische Wirkung gegen das besagte Transporterprotein eingesetzt wird und
c) die Verbindung zusätzlich auf Aktivität gegenüber dem Organismus oder der Zelle untersucht wird, der oder die das entsprechenden Transporter protein nicht produziert, um die Möglichkeit auszuschließen, daß die Verbindung auch gegen andere Mechanismen dieses Organismus oder der Zelle inhibitorisch wirkt, und schließlich
d) die gegen das Transporter protein aktive Verbindung auf ihre herbizide oder wachstumsregulatorische Aktivität gegen Pflanzen geprüft wird.

2. Verfahren gemäß Anspruch 1, dadurch gekennzeichnet, daß das Transporter protein ein Saccharosetransporter ist.

3. Verfahren gemäß Anspruch 1, dadurch gekennzeichnet, daß das Transporter protein ein Aminosäuretransporter ist.

4. Verfahren gemäß Anspruch 1, dadurch gekennzeichnet, daß das Transporter protein ein Ammoniumtransporter ist.

5. Verfahren gemäß den Ansprüchen 1 bis 4, dadurch gekennzeichnet, daß der transgene Organismus ein einzelliger Organismus ist.

6. Verfahren gemäß den Ansprüchen 1 bis 5, dadurch gekennzeichnet, daß der einzellige Organismus Bakterie, Pilz oder Hefe ist.

7. Verfahren gemäß den Ansprüchen 1 bis 5, dadurch gekennzeichnet, daß es sich bei den Zellen um pflanzliche Zellen handelt.

8. Verfahren gemäß den Ansprüchen 1 bis 5, dadurch gekennzeichnet, daß es sich bei den Zellen um tierische Zellen handelt.

9. Verfahren gemäß den Ansprüchen 1 bis 5, dadurch gekennzeichnet, daß es sich bei den Zellen um Oozyten handelt.

10. Verfahren gemäß den Ansprüchen 1 bis 5, dadurch gekennzeichnet, daß es sich bei den Zellen um Xenopus-Oozyten handelt.

## Claims

1. A method for identifying substances which have a potential herbicidal or growth-regulating action which arises due to inhibition or inactivation of a plant transport process, which comprises
a) initially preparing the transporter protein by heterologous expression of a DNA sequence which codes for this transport protein in a transgenic organism or transgenic cells, subsequently
b) employing this recombinant organism in its entirety or the transgenic cells for investigating a chemical compound for its inhibitory effect on said transporter protein, and
c) additionally investigating the compound for activity on the organism or cell which does not produce the corresponding transporter protein, in order to preclude the possibility that the compound also has an inhibitory effect on other mechanisms in this organism or cell, and finally
d) testing the compound which is active against the transporter protein for its herbicidal or growth-regulating activity on plants.

2. A method as claimed in claim 1, wherein the transporter protein is a sucrose transporter.

3. A method as claimed in claim 1, wherein the transporter protein is an amino-acid transporter.

4. A method as claimed in claim 1, wherein the transporter protein is an ammonium transporter.

5. A method as claimed in claims 1 to 4, wherein the transgenic organism is a unicellular organism.

6. A method as claimed in claims 1 to 5, wherein the unicellular organism is bacterium, fungus or yeast.

7. A method as claimed in claims 1 to 5, wherein the cells are plant cells.

8. A method as claimed in claims 1 to 5, wherein the cells are animal cells.

9. A method as claimed in claims 1 to 5, wherein the cells are oocytes.

10. A method as claimed in claims 1 to 5, wherein the cells are xenopus oocytes.

## Revendications

1. Procédé pour l'identification de substances qui possèdent une activité potentielle herbicide ou régulatrice de la croissance des plantes, qui se concrétise par l'empêchement ou l'inactivation d'un processus de transport végétal, caractérisé par le fait que
a) d'abord la protéine transporteuse est préparée par expression hétérologue d'une séquence d'ADN qui code pour cette protéine transporteuse, dans un organisme transgénique ou une cellule transgénique, puis
b) on utilise cet organisme recombinant dans sa totalité ou les cellules transgéniques pour l'examen d'un composé chimique pour son activité inhibitrice contre ladite protéine transporteuse et
c) on étudie le composé également quant à son activité vis-à-vis de l'organisme ou de la cellule qui ne produit pas la protéine transporteuse correspondante, pour exclure la possibilité que le composé ait une action inhibitrice également contre d'autres mécanismes de cet organisme ou de la cellule, et enfin
d) le composé actif contre la protéine transporteuse est testé vis-à-vis de plantes pour son action herbicide ou régulatrice de la croissance des plantes.

2. Procédé selon la revendication 1, caractérisé par le fait que la protéine transporteuse est un transporteur de saccharose.

3. Procédé selon la revendication 1, caractérisé par le fait que la protéine transporteuse est un transporteur d'acide aminé.

4. Procédé selon la revendication 1, caractérisé par le fait que la protéine transporteuse est un transporteur d'ammonium.

5. Procédé selon les revendications 1 à 4, caractérisé par le fait que l'organisme transgénique est un organisme unicellulaire.

6. Procédé selon les revendications 1 à 5, caractérisé par le fait que l'organisme unicellulaire est une bactérie, un champignon ou une levure.

7. Procédé selon les revendications 1 à 5, caractérisé par le fait que, en ce qui concerne les cellules, il s'agit de cellules végétales.

8. Procédé selon les revendications 1 à 5, caractérisé par le fait que, en ce qui concerne les cellules, il s'agit de cellules animales.

9. Procédé selon les revendications 1 à 5, caractérisé par le fait que, en ce qui concerne les cellules, il s'agit d'oocytes.

10. Procédé selon les revendications 1 à 5, caractérisé par le fait que, en ce qui concerne les cellules, il s'agit d'oocytes de xénopus.
